(19) Europäisches Patentamt
European Patent Office
Office européen des brevets

(11) Publication number: **0 113 501**
**B1**

(12) # EUROPEAN PATENT SPECIFICATION

(45) Date of publication of patent specification: **30.12.86**

(21) Application number: **83306182.3**

(22) Date of filing: **12.10.83**

(51) Int. Cl.[4]: **C 08 K 5/32,** C 07 C 79/16, C 08 L 23/06, C 08 L 77/00

(54) **Antioxidants for plastics.**

(30) Priority: **25.11.82 GB 8233681**

(43) Date of publication of application: **18.07.84 Bulletin 84/29**

(45) Publication of the grant of the patent: **30.12.86 Bulletin 86/52**

(84) Designated Contracting States: **BE DE FR GB IT LU NL**

(56) References cited:
**US-A-4 007 159**
**US-A-4 153 597**

**The file contains technical information submitted after the application was filed and not included in this specification**

(73) Proprietor: **EUROPEAN ATOMIC ENERGY COMMUNITY (EURATOM)**
**Batiment Jean Monnet Plateau du Kirchberg**
**Boîte Postale 1907**
**Luxembourg (LU)**

(72) Inventor: **Le Goff, Bernard**
**Jollenpad 20**
**Alkmaar (NL)**

(74) Representative: **Baverstock, Michael George Douglas et al**
**BOULT, WADE & TENNANT 27 Furnival Street**
**London, EC4A 1PQ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 113 501 B1

**Description**

The present invention relates to antioxidants for plastics materials.

The processing of plastics materials necessitates the presence of antioxidants mainly to capture peroxides, especially for thermal and light stabilisation. These additives are used in plastics materials for food packaging. During prolonged contact between foodstuff and packaging material, extraction of low molecular weight components can occur. Consequently, additives will migrate from the packaging material in packed foods, creating a health hazard for consumers and leading to a loss of stabilising properties in the plastics materials. Both features are undesirable and there is a need for additives, such as antioxidants, which do not have this tendency to migrate to the surface of the plastics materials.

The migration behaviour of phenolic antioxidants is influenced by molecular shape and size. In a first approximation, migration rates depend on the molecular weight of the migrating species. However, increasing the molecular weight is limited by the requirement that the additive be capable of diffusing slowly through the plastics materials in order to exert its function. In DE—A—2.544.014 the use of polar phenolic antioxidants to stabilise plastics materials has been described, these antioxidants being alkanoic acids and alkanoate salts containing at least two 3,5 dialkyl-4-hydroxy phenyl substituents.

We have found that an improved result can be obtained with polar phenolic antioxidants consisting of molecules, having an alkyl chain with two end groups, the first being an optionally-substituted phenolic group and the other a trinitrophenyl group.

One example of these compounds is 3-(3,5 di-tertiary-butyl-4-hydroxy phenyl)propyl 2,4,6 trinitrophenyl ether, which in this specification will be referred to as TNPP.

If these substances are added to a polymer matrix with electron-donating properties (for instance a polyamide or a polyvinylpyrrolidone), a charge-transfer complex will be formed. However, the equilibrium between complex and "free" species allows sufficient "mobile" antioxidant to be available to capture peroxides in the plastics materials. Consumption of the antioxidant will shift the equilibrium gradually towards the release of "free" antioxidant species and maintain a functional behaviour for an extended service life.

The formation of charge-transfer complexes considerably reduces the spontaneous migration of the additive. Losses due to extraction by the packed foodstuffs will diminish, resulting in a lower contamination of the food and an extended life-time of the plastics materials.

The additives have been tested in plastics materials, such as High Density Polyethylene (HDPE) and Polyamide (PA).

The following antioxidants have been synthesised and tested in different polymers:

A) 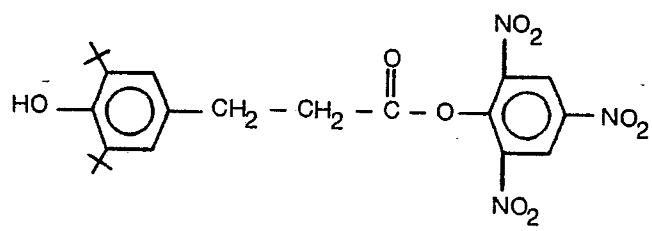

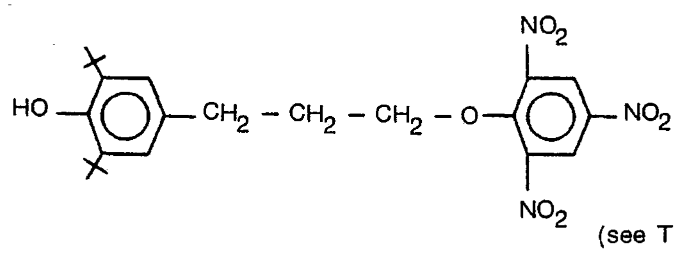

(see Table I)

in which the crosses in the phenyl group mean tertiary butyl groups.

Normally the amount of additive in the plastics materials is less than 1% by weight.

The migration values of the antioxidants used are represented in Tables I and II; in which BHT means the conventional 2,6 di-tertiary-butyl-4 methyl phenol and DMD means 5,5 dimethyl-2-(3,5-di-tertiary butyl-4-hydroxyphenyl)-1,3-dioxane.

TABLE 1

Migration values (as percentage of the initial concentration in the plastic) of 4 additives from pressed High Density Polyethylene into 3 test liquids after different storage conditions

| | Migrated Amount (weight %) in: HB307 | | Heptane | | Water/Ethanol (90/10) | |
|---|---|---|---|---|---|---|
| Storage time | 2 hrs | 10 days | 2 hrs | 10 days | 2 hours | 10 days |
| Storage temp. | 70°C | 40°C | 70°C | 40°C | 70°C | 40°C |
| Plastic/Additive | | | | | | |
| HDPE—BHT | 15.62 | 32.54 | 95.38 | 100.00 | 1.16 | 1.84 |
| HDPE—DMD | 9.15 | 21.62 | 87.86 | 100.00 | 2.27 | 2.98 |
| HDPE—TNPP | 5.59 | 12.01 | 73.95 | 56.62 | 0.62 | 0.38 |

TABLE II

Migration values of 3 additives from pressed Polyamide into 3 test liquids after different storage conditions

| | Migrated Amount (weight %) in: HB307 | | Heptane | | Water/Ethanol (90/10) | |
|---|---|---|---|---|---|---|
| Storage time | 2 hrs | 10 days | 2 hrs | 10 days | 2 hours | 10 days |
| Storage temp. | 70°C | 40°C | 70°C | 40°C | 70°C | 40°C |
| Plastic/Additive | | | | | | |
| PA—BHT | | 5.09 | | | | |
| PA—DMD | 0.09 | 0.16 | 0.12 | 0.25 | 6.24 | 20.89 |
| PA—NPP | n.d. | n.d. | n.d. | n.d. | 0.41 | 0.54 |

n.d. = not detected

**Claims**

1. A polar phenolic compound consisting of an alkylene unit with two end groups, the first being an optionally-substituted phenolic group and the other a trinitrophenyl group, the alkylene unit optionally including a divalent linking group which is an ester or ether group.

2. A compound as claimed in Claim 1 wherein the alkylene unit is a lower alkylene unit.

3. A compound as claimed in Claim 2 wherein the alkylene unit contains up to 3 carbon atoms.

4. A compound as claimed in any one of the preceding claims wherein the phenolic group is substituted with at least one tertiary butyl group.

5. 3-(3,5 di-tertiary-butyl-4-hydroxy phenyl) propyl 2,4,6 trinitrophenyl ether.

6. A plastics material including, as an antioxidant, at least one compound as claimed in any one of the preceding claims.

7. A high density polyethylene including, as an antioxidant at least one compound as claimed in any of Claims 1 to 5.

8. A polyamide including, as an antioxidant, at least one compound as claimed in any of Claims 1 to 5.

9. Use of a plastics material as claimed in any one of Claims 6 to 8 for food packaging.

**Patentansprüche**

1. Polare Phenolverbindung, bestehend aus einer Alkyleneinheit mit zwei Endgruppen, von denen die eine eine wahlweise substituierte Phenolgruppe und die andere eine Trinitrophenylgruppe ist, wobei die Alkyleneinheit wahlweise eine zweiwertige Bindegruppe, die eine Ester- oder Ethergruppe ist, enthält.

2. Verbindung nach Anspruch 1, wobei die Alkyleneinheit eine niedere Alkyleneinheit ist.

3. Verbindung nach Anspruch 2, wobei die Alkyleneinheit bis zu 3 Kohlenstoffatome enthält.

4. Verbindung nach einem der vorhergehenden Ansprüche, wobei die Phenolgruppe durch mindestens eine tertiäre Butylgruppe substituiert ist.

5. 3-(3,5-Di-tert.-butyl-4-hydroxyphenyl)propyl-2,4,6-trinitrophenylether.

6. Kunststoffmaterial, das als Antioxidationsmittel mindestens eine Verbindung gemäß den vorhergehenden Patentansprüchen enthält.

7. Hochdichtes Polyethylen, das als Antioxidationsmittel mindestens eine Verbindung gemäß einem der Ansprüche 1 bis 5 enthält.

8. Polyamid, das als Antioxidationsmittel mindestens eine Verbindung gemäß einem der Ansprüche 1 bis 5 enthält.

9. Verwendung eines Kunststoffmaterials nach einem der Ansprüche 6 bis 8 als Verpackungsmaterial für Nahrungsmittel.

**Revendications**

1. Un composé phénolique polaire consistant en un motif alkylène à deux groupes terminaux, le premier étant un groupe phénolique éventuellement substitué et l'autre un groupe trinitrophényle, le motif alkylène contenant éventuellement un chaînon divalent consistant en un groupe ester ou éther.

2. Un composé selon la revendication 1, dans lequel le motif alkylène est un motif alkylène inférieur.

3. Un composè selon la revendication 2, dans lequel le motif alkylène contient jusqu'à 3 atomes de carbone.

4. Un composé selon l'une quelconque des revendications qui précèdent dans lequel le groupe phénolique est substitué par au moins un groupe tert-butyle.

5. L'éther 3-(3,5-di-tert-butyl-4-hydroxyphényl)propyl-2,4,6-trinitrophénylique.

6. Une matière plastique contenant en tant qu'antioxydant au moins un composé selon l'une quelconque des revendications qui précèdent.

7. Un polyéthylène haute densité, contenant en tant qu'antioxydant au moins un composé selon l'une quelconque des revendications 1 à 5.

8. Un polyamide contenant en tant qu'antioxydant au moins un composé selon l'une quelconque des revendications 1 à 5.

9. L'utilisation d'une matière plastique selon l'une quelconque des revendications 6 à 8 pour l'emballage de produits alimentaires.